# EUROPEAN PATENT APPLICATION

(11) **EP 3 243 423 A1**
(43) Date of publication of application: **15.11.2017**
(21) Application number: 16857322.8
(22) Date of filing: 07.10.2016
(51) Int. Cl.: A61B 1/00, G02B 23/24

(54) **TIP HOOD FOR ENDOSCOPE**

(30) Priority: 22.10.2015 JP 2015208031
(71) Applicant: Olympus Corporation, Hachioji-shi Tokyo 192-8507 (JP)
(72) Inventor: SEKIGUCHI, Masahiko, Hachioji-shi Tokyo 192-8507 (JP)
(74) Representative: Schicker, Silvia
(86) International application number: PCT/JP2016/079986
(87) International publication number: WO 2017/069007

(57) **Abstract**

A distal end hood for an endoscope includes: a main body portion 51 formed in a cylindrical shape in a longitudinal axis direction N, wherein a distal end portion of an insertion portion of the endoscope is inserted inside through an opening 51k on a proximal end side in the longitudinal axis direction N, and the main body portion 51 is formed such that part of a distal end surface 51 s in the longitudinal axis direction N protrudes forward in the longitudinal axis direction; and a projection portion 52 provided on an outer peripheral surface 51 g of the main body portion 51 and formed in a rib shape extending toward a proximal end in the longitudinal axis direction N from a protrusion end surface 51 stm of a part 51 st of the distal end surface 51s protruding in the longitudinal axis direction N.

## Description

### Technical Field

The present invention relates to a distal end hood for an endoscope including a main body portion formed in a cylindrical shape in a longitudinal axis direction, wherein a distal end portion of an insertion portion of the endoscope is inserted inside through an opening on a proximal end side in the longitudinal axis direction.

### Background Art

In recent years, endoscopes have been widely used in a medical field and an industrial field. An elongated insertion portion of an endoscope used in the medical field can be inserted into a body cavity as a subject to observe an organ in the body cavity, and a treatment instrument inserted into an insertion channel of a treatment instrument included in the endoscope can be used as necessary to perform various treatments.

An elongated insertion portion of an endoscope used in the industrial field can be inserted into an object, such as a jet engine and a pipe of a factory, to perform inspections, such as observation and various treatments of a scratch, corrosion, or the like of a site to be examined in the object.

When the insertion portion of the endoscope is inserted into the subject or object to observe the site to be examined in the subject or object, a method using a distal end hood for an endoscope (hereinafter, simply called a hood) is well-known, the method preventing direct contact of the site to be examined and an observation lens provided on a distal end surface of a distal end portion of the insertion portion and maintaining a constant distance between the observation lens and the site to be examined to facilitate focusing the site to be examined.

More specifically, a method is well-known, in which a main body portion of the hood formed in a cylindrical shape in a longitudinal axis direction of the hood is installed on an outer periphery of the distal end portion of the insertion portion by inserting the distal end portion of the insertion portion into the main body portion through an opening on a proximal end side of the main body portion in the longitudinal axis direction (hereinafter, simply called a proximal end side), and the insertion portion is inserted into the subject or object in this state to observe the site to be examined.

However, when a wide-angle lens is used as the observation lens, there is a problem that a distal end surface of the main boy portion of the hood is photographed in a field of view range of the observation lens, because the distal end surface of the main body portion of the hood is positioned in front of the observation lens in the longitudinal axis direction (hereinafter, simply called in front) after the installation.

In view of the problem, Japanese Patent Application Laid-Open Publication No. 2003-290132 and Japanese Patent Application Laid-Open Publication No. 2003-93325 disclose so-called hoods without field-of-view vignetting, wherein a distal end surface of a main body portion of a hood has a wave shape in which a part blocking the field of view range of an observation lens is partially cut out, in other words, part of the distal end surface of the main body portion of the hood has a wave shape protruding forward.

By the way, the field of view range of the observation lens, that is, a shape of a field of view, a size of the field of view, a view angle, and the like, varies depending on a type of the observation lens (for example, a difference in a focal position) or a difference in a position of the observation lens on the distal end surface of the endoscope (for example, a difference in a distance from an outer peripheral surface of the endoscope to a center of the lens, a difference in a quadrant disposed based on a center axis of the distal end surface, and the like).

Therefore, considering that one type of hood is selectively installed on the distal end portions of the insertion portions of a plurality of types of endoscope, that is, considering a wide variety of field of view ranges of the observation lenses, the cutout formed on the distal end surface needs to be large to prevent the distal end surface of the main body portion of the hood from blocking the wide variety of field of view ranges of the observation lenses. Therefore, a part with a small contact area with the subject or object and protruding forward (hereinafter, called a protrusion part) is formed on the distal end surface of the main body portion of the hood. Note that the surface of the protrusion part contacting the subject or object in the distal end surface of the main body portion of the hood will be called a protrusion end surface, hereinafter.

If the protrusion end surface with a small contact area with the subject or object is formed on the distal end surface of the main body portion of the hood, the protrusion end surface with a small contact area may hurt a mucous membrane in a body cavity when, for example, the insertion portion is inserted into the body cavity while the hood is installed.

Therefore, a configuration is desired, wherein the protrusion part in the distal end surface of the main body portion of the hood can be formed as small as possible to prevent blocking the wide variety of field of view ranges of the observation lenses, and an area of the protrusion end surface can be secured and increased as much as possible.

When the hood is installed on the distal end portion of the insertion portion, an operator performs an operation of bringing an index as a reference of position in a rotation direction provided on the hood into line with, for example, an insertion channel of the treatment instrument opened on the distal end surface of the distal end portion of the insertion portion. However, when the protrusion part of the distal end surface of the main body portion of the hood blocks the field of view range of the observation lens after the installation, the operator makes an adjustment in some cases by rotating the main body portion of the hood and positioning the protrusion part outside of the field of view range of the observation lens.

Here, although the hood is generally configured from, for example, silicone rubber, there is a problem that rotating the main body portion of the hood is difficult when the hood is configured from rubber and is tightened and installed on the outer peripheral surface of the distal end portion of the insertion portion.

The present invention has been made in view of the circumstances and the problems, and an object of the present invention is to provide a distal end hood for an endoscope, in which a small size of a protrusion part is maintained on a distal end surface of a main body portion, a contact area between the protrusion part and a subject or object is secured and increased as much as possible, and the main body portion can be easily rotated.

### Disclosure of Invention

### Means for Solving the Problem

An aspect of the present invention provides a distal end hood for an endoscope including: a main body portion formed in a cylindrical shape in a longitudinal axis direction, wherein a distal end portion of an insertion portion of the endoscope is inserted inside through an opening on a proximal end side in the longitudinal axis direction, and the main body portion is formed such that part of a distal end surface in the longitudinal axis direction protrudes forward in the longitudinal axis direction; and a projection portion provided on an outer peripheral surface of the main body portion and formed in a rib shape extending toward a proximal end in the longitudinal axis direction from a protrusion end surface of a part of the distal end surface protruding in the longitudinal axis direction.

### Brief Description of the Drawings

Fig. 1 is a diagram showing, along with a hood, an appearance of an endoscope including an insertion portion, a distal end portion of which is inserted into the hood, according to the present embodiment;
Fig. 2 is a plan view showing a distal end surface of the distal end portion of the insertion portion of Fig. 1;
Fig. 3 is an enlarged perspective view of the hood of Fig. 1;
Fig. 4 is a side view of the hood of Fig. 3 as viewed from a IV direction in Fig. 3;
Fig, 5 is a top view of the hood of Fig. 3 as viewed from a V direction in Fig. 3; and
Fig. 6 is a side view of the hood showing a modification of a shape of a projection portion of Fig. 4.

### Best Mode for Carrying Out the Invention

Hereinafter, an embodiment of the present invention will be described with reference to the drawings. Note that the drawings are schematic drawings, and a relationship between a thickness and a width of each member, a ratio of the thickness of respective members, and the like are different from the reality. It is obvious that the relationship and the ratio of dimensions between the drawings are different in some parts of the drawings.

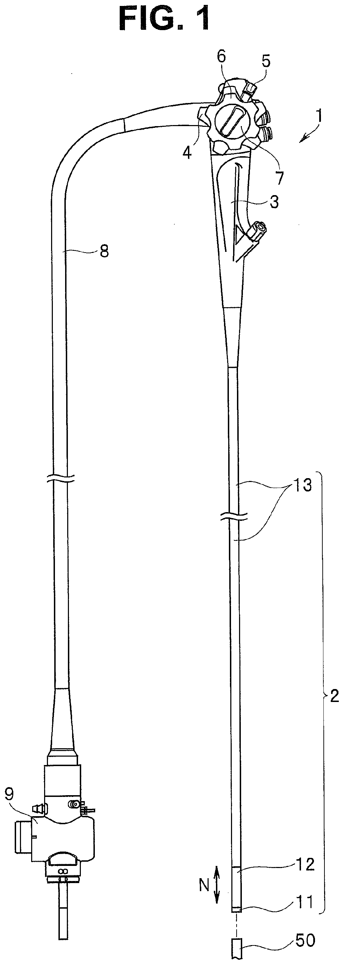

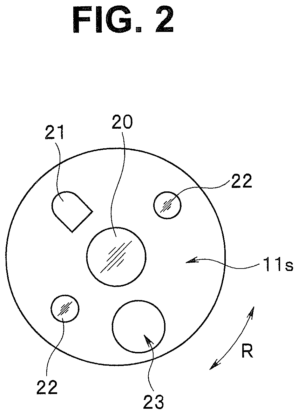

Fig. 1 is a diagram showing, along with a hood, an appearance of an endoscope including an insertion portion, a distal end portion of which is inserted into the hood, according to the present embodiment. Fig. 2 is a plan view showing a distal end surface of the distal end portion of the insertion portion of Fig. 1.

As shown in Fig. 1, main parts of an endoscope 1 include: an insertion portion 2 inserted into a subject or object; an operation portion 3 consecutively connected to a proximal end side of the insertion portion 2; a universal cord 8 extended from the operation portion 3; and a connector 9 provided on an extension end of the universal cord 8.

Note that the endoscope 1 is electrically connected to an external device, such as an image processing apparatus and an illumination apparatus, through the connector 9.

The operation portion 3 is provided with: an up-down bending operation knob 4 configured to bend a bending portion 12 described later of the insertion portion 2 in an up-down direction; and a left-right bending operation knob 6 configured to bend the bending portion 12 in a left-right direction.

The operation portion 3 is further provided with: a fixation lever 5 configured to fix a rotation position of the up-down bending operation knob 4; and a fixation knob 7 configured to fix a rotation position of the left-right bending operation knob 6.

The insertion portion 2 includes a distal end portion 11, the bending portion 12, and a flexible tube portion 13 in order from a distal end side and is formed in an elongated shape.

The bending portion 12 is bent, for example, in four directions of up, down, left, and right through rotation operation of the up-down bending operation knob 4 and the left-right bending operation knob 6 to change a field of view direction of an observation lens 20 provided on a distal end surface 11s of the distal end portion 11 and to improve an insertion property of the distal end portion 11 in the subject or object.

Furthermore, the flexible tube portion 13 is flexible and is consecutively connected to a proximal end side of the bending portion 12.

As shown in Fig. 2, the distal end surface 11s is provided with, in addition to the observation lens 20: a fluid supply nozzle 21 configured to supply a fluid to the observation lens 20; illumination lenses 22 configured to illuminate inside of the subject or object; an opening of an insertion channel 23 of a treatment instrument; and the like. Note that in place of the illumination lenses 22, light emitting elements, such as LEDs, may be provided on the distal end surface 11s.

In addition, the distal end portion 11 can be inserted into a hood 50. In other words, the hood 50 can be attached to and detached from an outer periphery of the distal end portion 11.

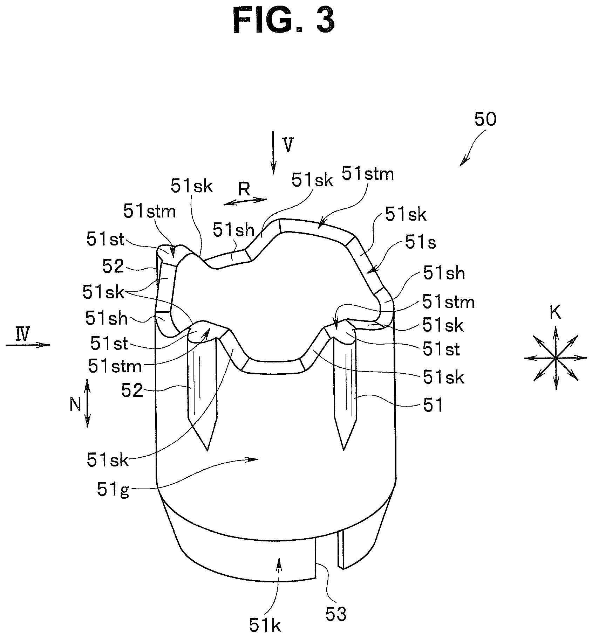

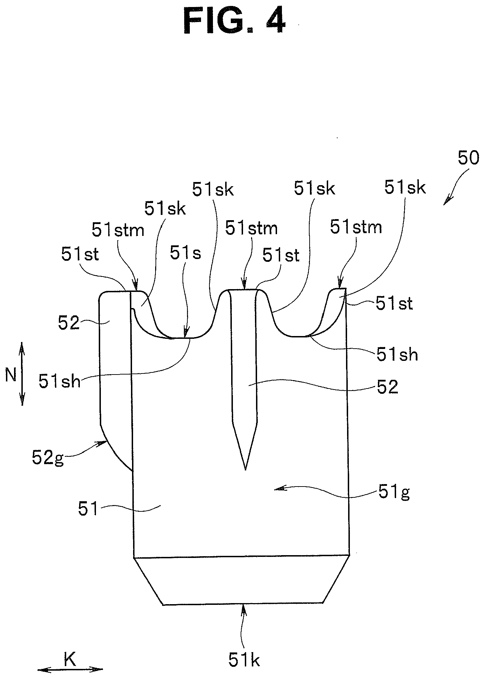

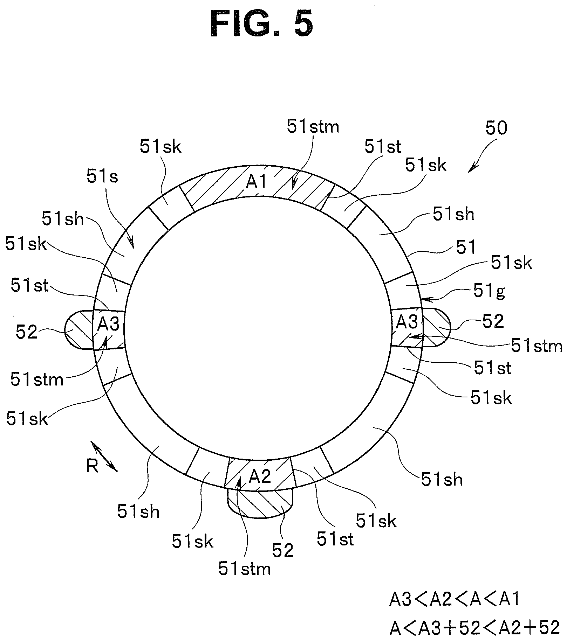

Next, a configuration of the hood 50 will be described with reference to Figs. 3 to 5. Fig. 3 is an enlarged perspective view of the hood of Fig. 1. Fig. 4 is a side view of the hood of Fig. 3 as viewed from a IV direction in Fig. 3. Fig. 5 is a top view of the hood of Fig. 3 as viewed from a V direction in Fig. 3.

As shown in Figs. 3 to 5, the hood 50 includes a main body portion 51 configured from, for example, silicone rubber and formed in a cylindrical shape along a longitudinal axis direction N.

When the hood 50 is installed on the outer periphery of the distal end portion 11, the distal end portion 11 of the insertion portion 2 is inserted into the main body portion 51 through an opening 51k on the distal end side.

After the installation of the hood 50, a distal end surface 51s of the main body portion 51 is positioned in front of the observation lens 20 provided on the distal end surface 11s although not shown.

Furthermore, an index 53 is formed on the proximal end side of the hood 50 as shown in Fig. 3. The index 53 is used for positioning of the hood 50 in a circumferential direction R with respect to the distal end portion 11 when the hood 50 is installed on the outer periphery of the distal end portion 11. For example, the index 53 is used such that the position in the circumferential direction R coincides with the insertion channel 23 of the treatment instrument opened on the distal end surface 11s.

Furthermore, the distal end surface 51s of the main body portion 51 has a wave shape, such that part of the distal end surface 51s, more specifically, a section blocking a field of view range of the observation lens 20, is cut out.

In other words, the distal end surface 51s of the main body portion 51 has a wave shape, such that part of the distal end surface 51s protrudes forward in the longitudinal axis direction N, and the other part is formed by concave portions 51sh.

Note that protrusion parts 51 st protruding forward in the distal end surface 51s are positioned outside of the field of view range of the observation lens 20. That is, the reason that the distal end surface 51s has the wave shape is that the distal end surface 51s is positioned in front of the observation lens 20 as described above, and the concave portions 51 sh prevent the blocking of the field of view range of the observation lens 20.

As shown in Figs. 3 to 5, a plurality of protrusion parts 51 st and concave portions 51 sh are formed in, for example, the circumferential direction R. For example, four protrusion parts 51st and four concave portions 51 sh are formed in the case illustrated in Figs. 3 to 5.

Note that the number of protrusion parts 51 st and concave portions 51 sh is not limited to four, and the number may be any number. In addition, the number may be one. Note that the respective protrusion parts 51 st and concave portions 51 sh are connected in the circumferential direction R through inclined surfaces 51sk.

Furthermore, the size of all protrusion parts 51 st in the circumferential direction R, that is, each area of protrusion end surface 51 stm, may not be the same, and areas A1 to A3 may be different in each protrusion end surface 51stm as shown in Fig. 5.

Here, as shown in Figs. 3 to 5, projection portions 52 formed in a rib shape extending toward the proximal end from the protrusion end surfaces 51stm of the respective protrusion parts 51 st are provided integrally with the main body portion 51 on an outer peripheral surface 51 g of the main body portion 51.

More specifically, the projection portions 52 are provided on the protrusion parts 51 st including the protrusion end surfaces 51 stm with areas smaller than a set area A among the plurality of protrusion parts 51 st.

Therefore, as shown in Fig. 5, the projection portions 52 are provided on the protrusion parts 51 st with the areas A2 and A3 smaller than the set area A (A3<A2<A) and are not provided on the protrusion parts 51 st with the area A1 greater than the set area A (A<A1).

In the present embodiment, each projection portion 52 has a shape linearly extending in the longitudinal axis direction N as shown in Figs. 3 and 4.

Note that although each projection portion 52 needs to have a shape extending toward the proximal end from each protrusion end surface 51stm, each projection portion 52 does not have to be formed in a shape extending up to the proximal end of the main body portion 51 in the longitudinal axis direction N as shown in Figs. 3 and 4.

Like the protrusion parts 51 st, each projection portion 52 is positioned outside of the field of view range of the observation lens 20 through each concave portion 51sh.

Furthermore, each projection portion 52 is formed in a shape in which a part closer to the proximal end is inclined inside in a radial direction K of the main body portion 51 as shown in Figs. 3 and 4. That is, each projection portion 52 includes an inclined surface 52g inclined inside in the radial direction K, on the part closer to the proximal end.

This is because the inclined surface 52g prevents the part of each projection portion 52 closer to the proximal end from blocking the field of view range of the observation lens 20 through each concave portion 52sh, and the inclined surface 52g prevents the part of each projection portion 52 closer to the proximal end from being caught inside of the subject or object when the insertion portion 2 provided with the hood 50 on the outer periphery of the distal end portion 11 is shaken and inserted into the subject or object.

Furthermore, the projection portion 52 extending toward the proximal end from each protrusion end surface 51stm is provided on each protrusion part 51 st including the protrusion end surface 51stm smaller than the set area A. This secures the contact area between each protrusion part 51 st, which includes the protrusion end surface 51stm with the area smaller than the set area A, and the subject or object increased by the projection portion 52 (A<A3+52<A2+52).

Note that other configurations of the hood 50 are the same as configurations of a conventional hood, and the configurations will not be described.

In this way, the present embodiment has illustrated that the distal end surface 51s of the main body portion 51 of the hood 50 is formed in a wave shape including the plurality of protrusion parts 51 st in the circumferential direction R such that the distal end surface 51s does not block the field of view range of the observation lens 20.

Furthermore, the present embodiment has illustrated that the projection portion 52 extending toward the proximal end from each protrusion end surface 51stm is provided on each protrusion part 51 st, the protrusion end surface 51stm of which has the area A2 or A3 smaller than the set area A, among the plurality of protrusion parts 51 st.

According to this, on the protrusion end surfaces 51 stm with the areas A2 and A3 smaller than the set area A, the contact with the subject or object may hurt, for example, a mucous membrane in a body cavity as described above. However, according to the configuration of the present embodiment, the projection portion 52 extending toward the proximal end from each protrusion end surface 51 stm is provided on each protrusion end surface 51 stm with the area A2 or A3 smaller than the set area A. As a result, the contact area between the distal end surface 51s and the subject or object is increased by the projection portions 52, and the pressure to the subject or object is dispersed. Therefore, for example, a mucous membrane in a body cavity is not hurt.

Furthermore, each projection portion 52 is formed in a rib shape. As a result, even when each protrusion part 51 st or each projection portion 52 blocks the field of view range of the observation lens 20, and the hood 50 needs to be rotated in the circumferential direction to move each protrusion part 51st and each projection portion 52 to the outside of the field of view range of the observation lens 20 as described above, the operator can hook the fingers on each projection portion 52 to perform the rotation operation, and the hood 50 can be easily rotated. Therefore, the positioning operation of the hood 50 in the circumferential direction R can be easily performed.

As a result, the hood 50 can be provided, in which the small size of the protrusion parts 51 st is maintained on the distal end surface 51 s of the main body portion 51, the contact area between the protrusion parts 51 st and the subject or object is secured and increased as much as possible, and the main body portion 51 can be easily rotated.

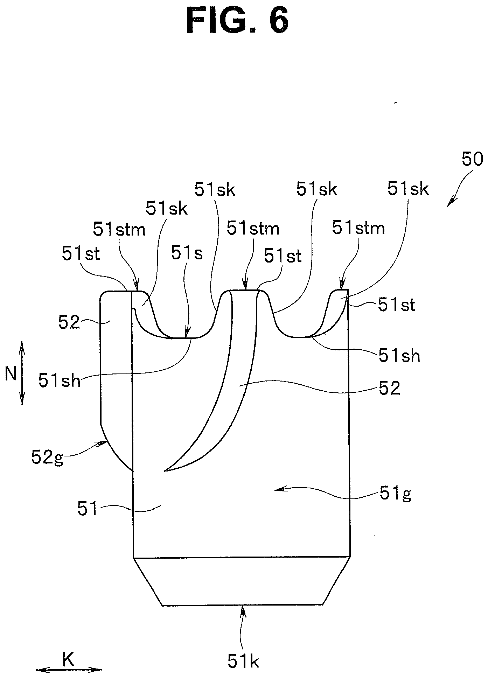

Note that a modification will be illustrated below with reference to Fig. 6. Fig. 6 is a side view of the hood illustrating a modification of the shape of the projection portions of Fig. 4.

In the present embodiment described above, the projection portions 52 have a linear shape extending in the longitudinal axis direction N.

Regardless of this, the projection portions 52 may be formed in a bent shape extended and inclined with respect to the longitudinal axis direction N as shown in Fig. 6.

Note that the shape of the projection portions 52 shown in Fig. 6 is just an example, and the shape can be a spiral shape or any other shapes as long as the shape is inclined with respect to the longitudinal axis direction N.

According to the configuration, the operator can more easily hook the fingers on the projection portions 52 in rotating the hood 50, and the hood 50 can be easily rotated. Note that the other advantageous effects are the same as in the present embodiment described above.

The present application is filed on the basis of claiming the benefit of priority from Japanese Patent Application No. 2015-208031, filed on October 22, 2015 in Japan, and the content is incorporated in the present specification, the claims, and the drawings by reference.

## Claims

1. A distal end hood for an endoscope comprising:
a main body portion formed in a cylindrical shape in a longitudinal axis direction, wherein a distal end portion of an insertion portion of the endoscope is inserted inside through an opening on a proximal end side in the longitudinal axis direction, and the main body portion is formed such that part of a distal end surface in the longitudinal axis direction protrudes forward in the longitudinal axis direction; and
a projection portion provided on an outer peripheral surface of the main body portion and formed in a rib shape extending toward a proximal end in the longitudinal axis direction from a protrusion end surface of a part of the distal end surface protruding in the longitudinal axis direction.

2. The distal end hood for an endoscope according to claim 1, wherein
the projection portion extends in the longitudinal axis direction.

3. The distal end hood for an endoscope according to claim 1, wherein
the projection portion is extended and inclined with respect to the longitudinal axis direction.

4. The distal end hood for an endoscope according to any one of claims 1 to 3, wherein
a part of the distal end surface of the main body portion protruding in the longitudinal axis direction and the projection portion are positioned outside of a field of view range of the endoscope.

5. The distal end hood for an endoscope according to any one of claims 1 to 4, wherein
the part of the distal end surface of the main body portion protruding in the longitudinal axis direction is formed in plurality in a circumferential direction of the main body portion, and
the projection portion is formed on the part with the protrusion end surface smaller than a set area among the plurality of parts of the distal end surface of the main body portion protruding in the longitudinal axis direction.

6. The distal end hood for an endoscope according to any one of claims 1 to 5, wherein
the projection portion is formed in a shape such that a section on the proximal end side in the longitudinal axis direction is inclined inward in a radial direction of the main body portion.
